# EUROPEAN PATENT APPLICATION

(11) **EP 1 160 320 A1**
(43) Date of publication of application: **05.12.2001**
(21) Application number: 00401235.7
(22) Date of filing: 05.05.2000
(51) Int. Cl.: C12N 15/12, C07K 14/47, A61K 48/00, A61K 38/17, G01N 33/68, G01N 33/53

(54) **Use of hTAFII80 and isoforms thereof for inducing apoptosis**

(71) Applicant: INSTITUT NATIONAL DE LA SANTE ET DE LA RECHERCHE MEDICALE (INSERM), 75013 Paris (FR); CENTRE NATIONAL DE LA RECHERCHE SCIENTIFIQUE (CNRS), 75794 Paris Cedex 16 (FR)
(72) Inventor: Bell, Brendan, 67100 Strasbourg (FR); Tora, Laszlo, 67880 Krautergersheim (FR)
(74) Representative: Ahner, Francis

(57) **Abstract**

The present invention relates to a hTAF_{II}80 isoform, namely hTAF_{II}80δ, which comprises a 10 amino acid deletion, said isoform being capable of inducing apoptosis in mammalian cells. It also concerns the use of a compound for inhibiting or activating the expression or activity of hTAF_{II}80 protein or isoforms thereof for treating diseases involving cell apoptosis, notably nerve cell degeneration, such as Alzheimer disease and Parkinson disease, cancer and AIDS.

## Description

The present invention relates to a hTAF_{II}80 isoform, namely hTAF_{II}80δ, which comprises a 10 amino acid deletion, said isoform being capable of inducing apoptosis in mammalian cells. It also concerns the use of a compound for inhibiting or activating the expression or activity of hTAF_{II}80 protein or isoforms thereof for treating diseases involving cell apoptosis, notably nerve cell degeneration, such as Alzheimer disease and Parkinson disease, cancer and AIDS.

Apoptosis, or active cell suicide, plays an essential role in normal tissue homeostasis and development of multicellular organisms (White E. et al, 1996). The initiation of apoptosis is associated with changes in gene expression (Jehn et al, 1997), but the connections between death inducing signals and the regulation of gene transcription remain largely obscure. It is therefore of a great interest to identify proteins involved in the regulation that triggers cell suicide, which could ultimately lead to the development of new treatments for diseases such as cancer, retrovirus infection and neurodegenerative syndromes.

hTAF_{II}80 also named hTAF_{II}70 (Weinzierl et al, 1993 and Hisatake et al, 1995) is a subunit of TF_{II}D, a multiprotein complex composed of TATA-binding protein (TBP) and more than 12 TBP-associated factors (TAFs) (Green et al, 2000 and Albright et al, 2000). TF_{II}D plays a key role in the initiation of RNA polymerase II (Pol II) transcription by nucleating the formation of the pre-initiation complex (PIC) at core promoter elements (Burley et al, 1996 and Bell et al, 1999) and it has been suggested that it could be involved in apoptosis regulation (Sekiguchi et al, 1995 and Metzger et al, 1999). hTAF_{II}80 contains a histone H4-like histone fold that interacts with its histone H3-like partner hTAF_{II}31 (Hisatake et al, 1995, Xie et al, 1996 and Bando et al, 1997).

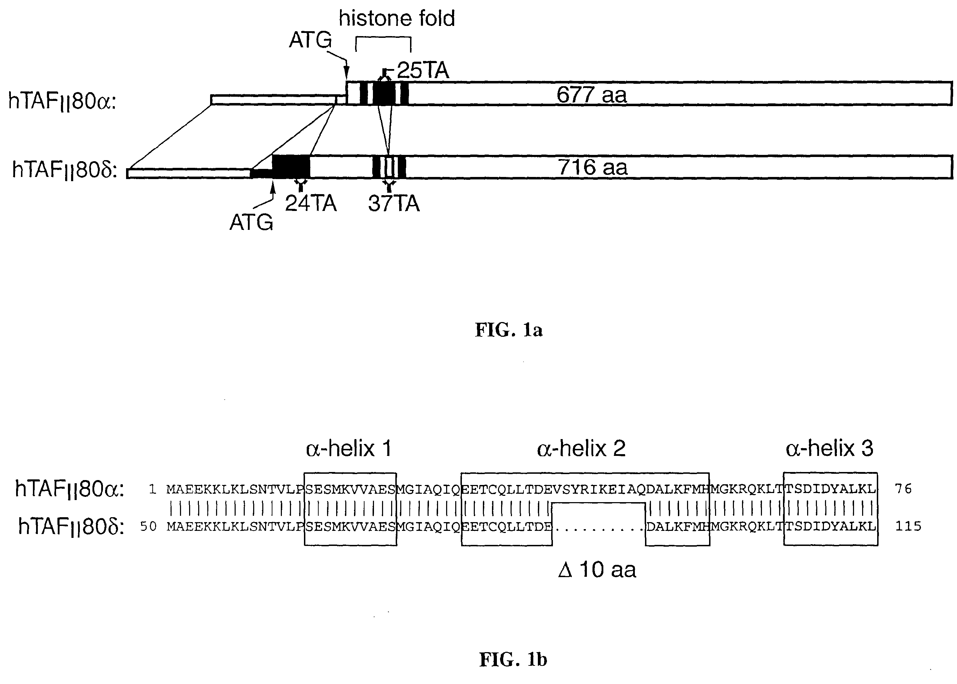

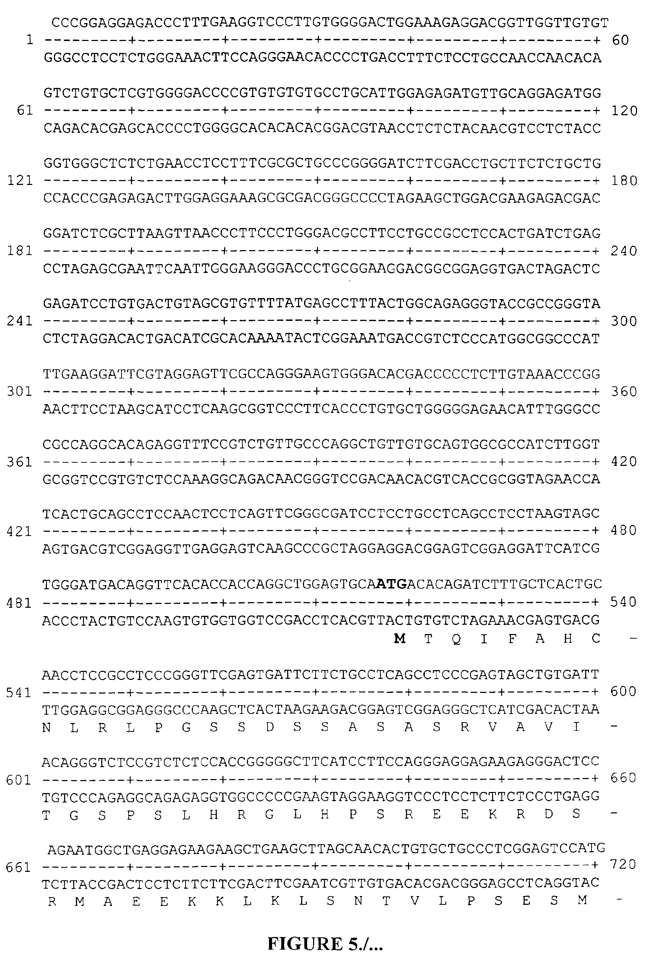

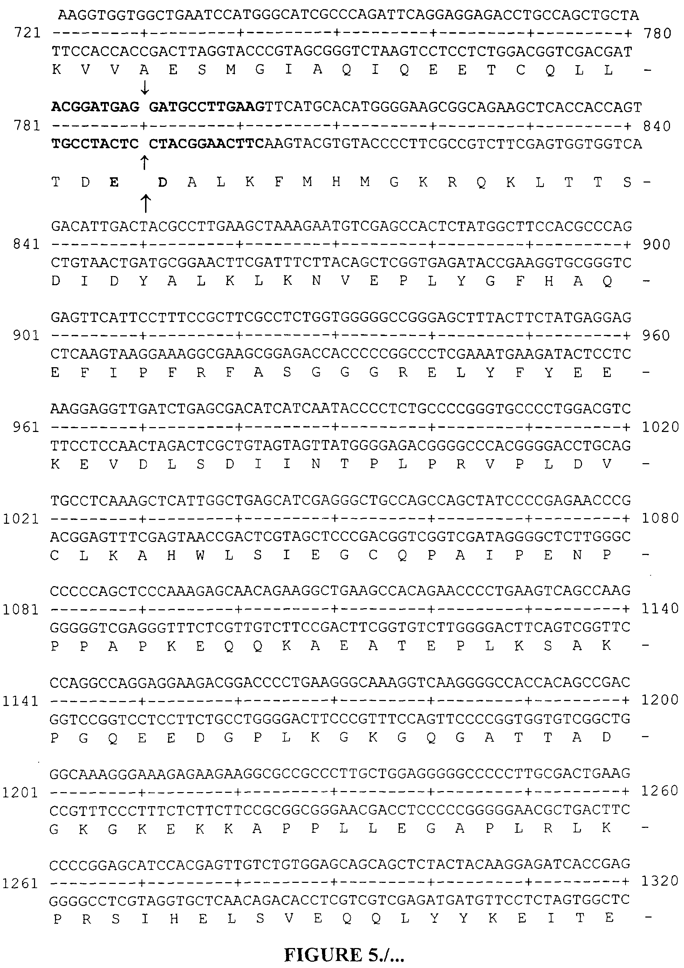

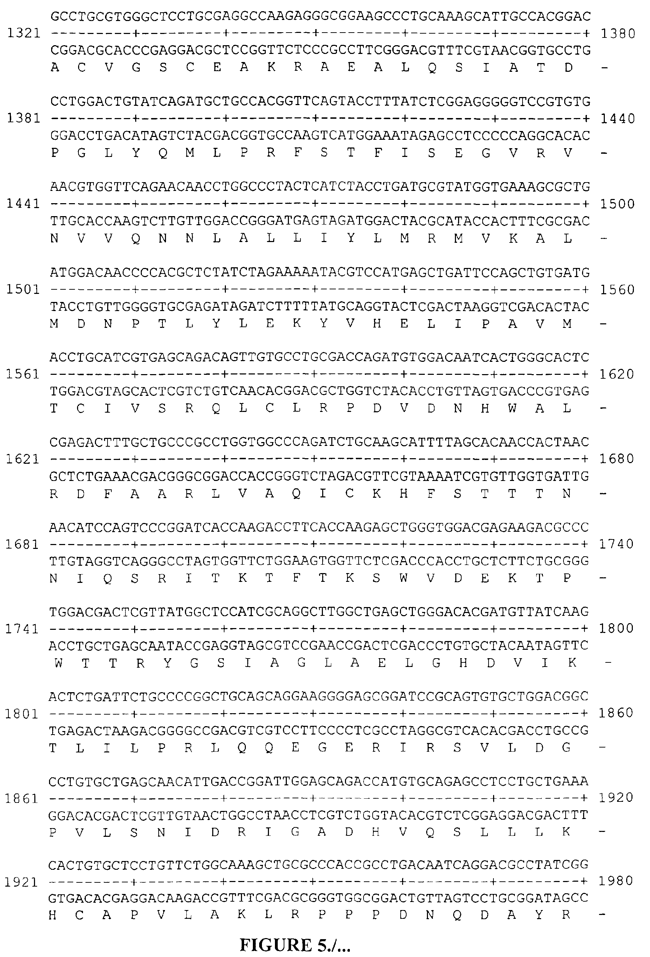

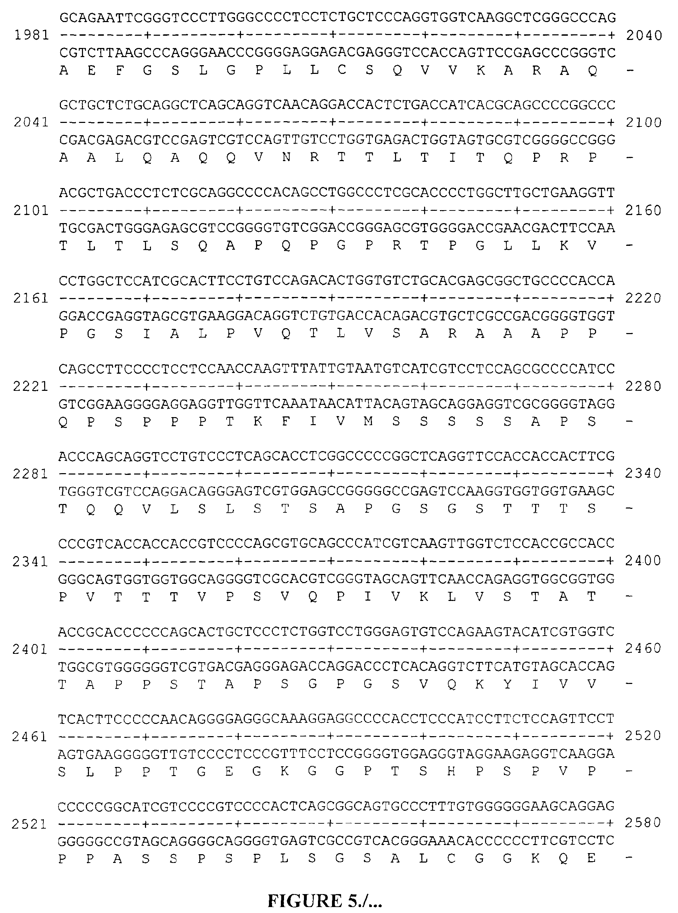

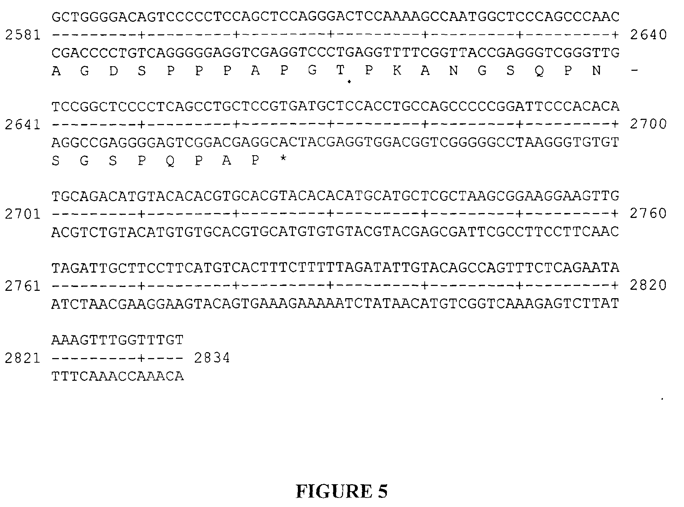

In connection with the present invention, we cloned a cDNA coding for a novel isoform of hTAF_{II}80. This isoform will be hereinafter referred to as hTAF_{II}80δ to distinguish it from previously described isoforms of hTAF_{II}80 (hTAF_{II}80α, β, and γ) Weinzierl et al, 1993. hTAF_{II}80δ has the remarkable feature of a 10 amino acid deletion in the centre of α-helix 2 of its histone fold motif (Fig. 1 and 5). The amino acids removed by this deletion include many of the residues implicated in dimerization contacts with hTAF_{II}31 by crystallographic studies of hTAF_{II}80's *Drosophila* homolog, dTAF_{II}60/62 (Wie et al, 1996). In addition, the hTAF_{II}80δ cDNA encodes a predicted unique 49 amino acid tail at the N-terminus of the open reading frame (Fig. 1a). Surprisingly, we found that hTAF_{II}80δ overexpression induces apoptosis in different cell lines.
Accumulating biochemical and genetic evidence supports the existence of functionally distinct TFIIDs within cells (Bell et al, 1999). The present findings provide the first evidence implying that TFIID composition in living cells is dynamic, and suggest that cells can employ changes in the subunit composition of TFIID to alter cell metabolism in response to changing growth conditions. Our results also identify hTAF_{II}80δ as an important part of a novel signalling pathway that acts as an interface between apoptotic signals and the Pol II transcriptional machinery. Cell death-inducing signals induce the novel TFIIDπ complex, which in turn reprogrammes Pol II transcription so that the balance between transcription of pro- and anti-apoptotic genes favors cell death.

### Description

In a first embodiment, the present invention is aimed at a polypeptide comprising a sequence having at least 80%, 90%, 95% or 99% identity with hTAF_{II}80δ of the sequence SEQ ID N°1 characterized in that it has a 10 amino acids deletion at position 43 to 52 of SEQ ID N°3 (hTAF_{II}80), said polypeptide being capable of inducing apoptosis when expressed in mammalian cells.
Preferably, said polypeptide comprises the sequence SEQ ID N°1 corresponding to the hTAF_{II}80δ, which is also depicted at figure 5.

Nucleic sequence or nucleic acid is understood to mean an isolated natural, or a synthetic, DNA and/or RNA fragment comprising, or otherwise, non natural nucleotides, designating a precise succession of nucleotides, modified or otherwise, allowing a fragment, a segment or a region of a nucleic acid to be defined.
In frame with the invention, "percent identity" means the percentage of identical nucleotides between two sequences when compared according to the best alignments, this percentage being statistical and the differences between the two sequences being at random and on the total length of the sequences. Optimal alignments of sequences can be achieved by means of the algorithm of Smith Waterman (1981) [Ad. App. Math. 2 : 482], with the algorithm for local homology described in Neddleman and Wunsch (1970) [J. Mol. Biol. 48 : 443], with the similarity search method described by Lipman (1988) [Proc. Natl. Acad. Sci. USA 85 : 2444], with softwares using GAP, BESTFIT, FASTA and TFASTA algorithms available in Wisconsin Genetics Software Package, Genetics Computer Group, 575 Science Dr., Madison, WI and DNASIS,Version 2.5 for Windows; Hitachi Software Engineering Co., Ltd, South San Francisco, CA, by using the standard parameters described in the manufacturer brochure.
Another possibility is to use the BLAST or FASTDB programs available at WWW.ncbi.nlm.nih.gov with the following parameters "Mismatch penalty 1.00; Gap Penalty 1.00; Gap Size Penalty 0.33; joining penalty 30.0. These algorithms are displayed in Current Methods in Sequencing and synthesis Methods and Applications, pages 127-149, 1988, Ala R. Liss, Inc".

The invention also relates to an isolated nucleic acid sequence coding for a polypeptide mentioned above and more particularly to an isolated nucleic acid sequence coding for hTAF_{II}80δ having the sequence SEQ ID No. 1, said hTAF_{II}80δ protein being an isoform of hTAF_{II}80 comprising a deletion of the 10 amino acids at position 43 to 52 of hTAF_{II}80 and being capable of inducing apoptosis when expressed in mammal cells. Preferably, said nucleic acid sequence described above has a sequence comprising the sequence SEQ ID N°2.
Complementary sequences of the nucleic sequence according to the invention is another embodiment as well as an isolated nucleic acid sequence, characterized in that it is selected from the group consisting of:
a) a fragment of nucleic acid sequence according to the invention comprising at least 12, 15, 20, or 25 bases present at the junction of the deletion, more particularly a sequence encompassing G789 and G790 of the sequence depicted at figure 5,
b) a nucleic acid sequence capable of hybridizing specifically with the nucleic acid sequence as defined in a) and comprising at least 12, 15, 20, or 25 bases.
So, the invention deals with such a nucleic acid sequence as a primer or a probe.
Preferably, said sequences are characterized in that they comprise a sequence selected from the group consisting of sequences SEQ ID No. 4 (TGAGGATG) and SEQ ID No 5 (CATCCTCA).

Another aspect of the invention relates to a cloning and/or expression vector comprising a nucleic acid sequence described above. This vector can further comprise the elements allowing the expression and/or secretion of the said sequences in a host cell. Said host cell transformed by this vector is an eukaryotic or a prokaryotic cell.
The present invention also concerns a mammal, except human, characterized in that it comprises said cell.
The said vectors will preferably comprise a promoter, signals for initiation and termination of translation, as well as appropriate regions for regulation of transcription. They must be able to be stably maintained in the cell and may optionally possess particular signals specifying the secretion of the translated protein.
These different control signals are chosen according to the cellular host used. To this end, the nucleic acid sequences according to the invention may be inserted into autonomously replicating vectors inside the chosen host, or integrative vectors of the chosen host. Among the autonomously replicating systems, there will be preferably used according to the host cell, systems of the plasmid or viral type, it being possible for the viral vectors to be in particular adenoviruses, retroviruses, pox viruses or herpes viruses. One skilled in the art knows the technologies which can be used for each of these systems.
When the integration of the sequence into the chromosomes of the host cell is desired, it will be possible to use, for example, systems of the plasmid or viral type; such viruses will be, for example, retroviruses.
Such vectors will be prepared according to the methods commonly used by the man skilled in the art, and the clones resulting therefrom may be introduced into an appropriate host by standard methods such as, for example, lipofection, electroporation or heat shock.
Among the cells which can be transformed, there may of course be mentioned bacterial cells but also yeast cells, animal cells, in particular mammalian cell cultures, and in particular Chinese hamster ovary cells (CHO), but also insect cells in which it is possible to use methods using baculoviruses.
Among the animal models more particularly of interest here, there are:
- transgenic animals, preferably mice, overexpressing hTAF_{II}80δ. The mice can be obtained by transfection of multiple copies of the hTAF_{II}80δ gene under the control of a strong promoter of an ubiquitous nature, or selective for a type of tissue.
- animals (preferably rats, rabbits, mice) over-expressing hTAF_{II}80δ gene, after viral transcription or gene therapy.
- transgenic animals made deficient in TAF_{II}80δ gene by inactivation with the aid of the LOXP/CRE recombinase system or any other system for inactivating the expression of a gene of the animal.

Unless stated otherwise in the description below, the expression "hTAF_{II}80 isoforms" means hTAF_{II}80α, β, γ and δ.

Another aspect of the invention deals with Mono- or polyclonal antibodies or fragments thereof, chimeric or immunoconjugated antibodies, characterized in that they are capable of specifically recognizing a polypeptide as mentioned above. It also deals with a method for the diagnosis of a pathology associated with the expression or the lack of expression of hTAF_{II}80, hTAF_{II}80 isoforms, notably hTAF_{II}80δ or correlated with an abnormal expression of a polypeptide having the sequence SEQ ID N°1 and/or SEQ ID N°3, characterized in that one or more antibodies according to claim 14 is(are) brought into contact with the biological material to be tested, under conditions allowing the possible formation of specific immunological complexes between the said polypeptide and the said antibody or antibodies, and in that the immunological complexes that may be formed are detected.
The specific monoclonal antibodies may be obtained according to the conventional hybridoma culture method.
The antibodies according to the invention are, for example, chimeric antibodies, humanized antibodies, Fab or F(ab')2 fragments. They may also be in the form of immunoconjugates or of labeled antibodies so as to obtain a detectable and/or quantifiable signal.

In another embodiment, the invention concerns a method for the determination of an allelic variability or a loss of heterozygosity, characterized in that it uses a nucleic acid sequence as depicted above.
Allele or allelic variant will be understood to mean the natural mutated sequences corresponding to polymorphisms present in human beings and, in particular, to polymorphisms which can lead to the onset and/or to the development of the risks of pathology or pathogenesis associated with apoptosis.

Another embodiment deals with a method for determining if a subject is at increased risk of developing a pathology associated with the expression or the lack of expression of hTAF_{II}80, hTAF_{II}80 isoforms, notably hTAF_{II}80δ, said method comprising the steps consisting of:
a) collecting a biological sample containing genomic DNA or RNA from the subject;
b) testing for the presence of mutations, polymorphisms, or determining the sequence, or length thereof, of a fragment of said DNA or RNA susceptible of containing a mutation or a polymorphism associated with the risk of developing cancer and/or a pathology associated with the expression of hTAF_{II}80, hTAF_{II}80 isoforms, notably hTAF_{II}80δ, said fragment being amplified by polymerase chain reaction with a set of primers able to hybridize to a hTAF_{II}80, or isoforms thereof, sequence, for example primers derived from SEQ ID N°1 and/or SEQ ID N°3, such as the nucleic acid according to one of the claims 7 to 9;
c) determining whether or not the subject is at increased risk of developing a pathology associated with the expression or the lack of expression of hTAF_{II}80, hTAF_{II}80 isoforms, notably hTAF_{II}80δ by observing if the sequence of said fragment of DNA or RNA contains a mutation or polymorphism, the presence of said mutation or polymorphism being an indication that said subject is at increased risk of developing cancer and/or said pathology.
Preferably said pathologies are selected from the group consisting of cell apoptosis, notably nerve cell degeneration, such as the Alzheimer disease and Parkinson disease, cancer and AIDS.
Among the methods for the determination of an allelic variability, a mutation, a deletion, a loss of heterozygocity or a genetic abnormality, the methods comprising at least one stage for the so-called PCR (polymerase chain reaction) or PCR-like amplification of the target sequence according to the invention likely to exhibit an abnormality with the aid of a pair of primers of nucleotide sequences according to the invention are preferred. The amplified products may be treated with the aid of an appropriate restriction enzyme before carrying out the detection or assay of the targeted product.
PCR-like will be understood to mean all methods using direct or indirect reproductions of nucleic acid sequences, or alternatively in which the labeling systems have been amplified, these techniques are of course known, in general they involve the amplification of DNA by a polymerase; when the original sample is an RNA, it is advisable to carry out a reverse transcription beforehand. There are currently a great number of methods allowing this amplification, for example the so-called NASBA "Nucleic Acid Sequence Based Amplification", TAS "Transcription based Amplification System", LCR "Ligase Chain Reaction", "Endo Run Amplification" (ERA), "Cycling Probe Reaction" (CPR), and SDA "Strand Displacement Amplification", methods well known to persons skilled in the art.
In another embodiment, the invention is aimed at a kit for diagnostic or for determining if a subject is at increased risk of developing cancer and/or a pathology associated with the expression or the lack of expression of hTAF_{II}80, hTAF_{II}80 isoforms, notably hTAF_{II}80δ, comprising at least one pair of primers capable of amplifying a fragment of genomic DNA or RNA encoding the protein hTAF_{II}80, hTAF_{II}80 isoforms, notably hTAF_{II}80δ, preferably primers being chosen among primers derived from SEQ ID N°1 such as a fragment of nucleic acid sequence according to the invention comprising at least 12, 15, 20, or 25 consecutive bases specific to hTAF_{II}80δ present for example at the junction of the deletion, more particularly a sequence encompassing G789 and G790 of the sequence depicted at figure 5.
A kit according to the invention may alternatively comprise at least one monoclonal antibody specifically recognizing hTAF_{II}80δ. With a kit comprising a specific hTAF_{II}80δ antibody and a general hTAF_{II}80 antibody, it is possible to calculate the hTAF_{II}80δ / total hTAF_{II}80 ratio and assess the ability of a cell to respond to apoptosis stimuli. Such kits can be used in a RIA or ELISA test.

Another aspect of the invention relates to the use of a cell, mammal or vector described above for assessing the expression and/or the activity of the protein hTAF_{II}80, hTAF_{II}80 isoforms, notably hTAF_{II}80δ, for studying the direct or indirect interactions between said proteins with other TAF proteins, more particularly with hTAF_{II}31, and for selecting compounds that modulate the activity and/or expression of said protein.

Such cell, mammal or vector are preferably used for screening compounds capable of interacting directly or indirectly with hTAF_{II}80 or hTAF_{II}80 isoforms, notably with hTAF_{II}80δ, and/or capable of modulating the expression or the activity of said proteins.
So, the invention is aimed at a process for selecting compounds capable of modulating the activity of hTAF_{II}80 and/or hTAF_{II}80 isoforms, notably hTAF_{II}80δ characterized in that it comprises the step consisting of:
a) overexpressing hTAF_{II}80 or hTAF_{II}80 isoforms, notably hTAF_{II}80δ in host cells,
b) growing said cells in a medium containing at least one compound to be tested,
c) measuring the rate of apoptosis compared to cells grown in compound-free medium,
d) selecting compounds that reduce or increase significantly the rate of apoptosis.
   Yet, another aspect of the invention relates to compounds obtainable by said process.
   Said compounds are preferably characterized in that:
   - they decrease significantly the level of expression of hTAF_{II}80 or hTAF_{II}80 isoforms, notably hTAF_{II}80δ protein expression;
   - they increase by at least 50% the rate of apoptosis induced by the expression of said proteins; or
   - they decrease by at least 50% the rate of apoptosis induced by the expression of said proteins.

Compound according to one of claims 23 and 24, characterized in that it is chosen from:
a) an antibody according to the invention;
b) a peptide or a peptide like molecule;
c) a vector according to the invention;
d) a sense or anti-sense nucleic comprising a sequence able to hybridize to a nucleic acid according to the invention; or
e) a chemical.

A preferred aspect of the invention is aimed at the use of a compound mentioned above for preparing a drug, and more particularly for preparing a drug for treating diseases involving cell apoptosis, notably nerve cell degeneration, such as Alzheimer disease and Parkinson disease, for treating cancer or AIDS.

The present invention also concerns the use of a nucleic acid sequence coding for a hTAF_{II}80 protein or isoforms thereof, such as hTAF_{II}80α and δ, for inducing apoptosis, the use of a vector expressing a hTAF_{II}80 protein or isoforms thereof, such as hTAF_{II}80α and δ, for inducing apoptosis.
In this regard, it deals with the use of a fragment of at least 12, 15, 20 or 25 bases of a sequence derived from a nucleic acid sequence coding for a hTAF_{II}80 protein or isoforms thereof, such as hTAF_{II}80α and δ, as an anti-sense oligonucleotide for inhibiting apoptosis in a cell and with the use of said fragment as a probe or a primer for the diagnostic of cell apoptosis, notably nerve cells degeneration, such as the Alzheimer disease and Parkinson disease; cancer and AIDS or for determining if a subject is at increased risk of developing such diseases.

The invention is also directed to the use of a compound capable of inhibiting or activating the expression or activity of hTAF_{II}80 protein or isoforms thereof, such as hTAF_{II}80α and δ, for preparing a drug as well as to the use of a compound capable of interfering directly or indirectly with the hTAF_{II}80 protein or isoforms thereof interaction with other transcription associated factor proteins, preferably with other hTAFII proteins, such as hTAF_{II}31, for preparing a drug.
Said drug is particularly useful for treating diseases involving cell apoptosis, notably nerve cellss degeneration, such as Alzheimer disease and Parkinson disease; cancer and AIDS.

### Legends to Figures

### Figure 1: Isolation of a cDNA encoding a novel isoform of hTAF_{II}80.

a, Schematic representation of the cDNAs of two putative splice variants of hTAF_{II}80, and the open reading frames of the two isoforms they encode, hTAF_{II}80α and hTAF_{II}80δ. Narrow rectangles represent cDNA and thick rectangles indicate protein sequences. An inverted Alu cassette inserted into hTAF_{II}80δ is indicated by black shading, as are the α-helices of the histone fold motif. Start codons (ATG) are indicated with arrows and the epitopes used for antibody production are indicated by their names. **b**, A magnified view of the histone fold region shown in panel **a**, showing the primary structure of the histone homology regions of hTAF_{II}80α and hTAF_{II}80δ, respectively. aa: amino acid.

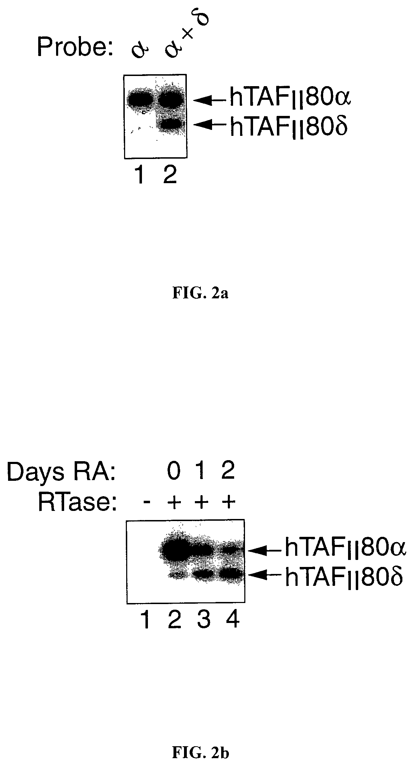

### Figure 2: Apoptotic induction of hTAF_{II}80δ expression.

**a,** RT-PCR was used to detect the mRNAs encoding either hTAF_{II}80δ or hTAF_{II}80α in HeLa cells. RT-PCR products were transferred to nylon membranes and revealed by hybridization with radiolabelled oligonucleotide probes specific for all hTAF_{II}80 isoforms except hTAF_{II}80δ (lane 1) or probes for all known hTAF_{II}80 isoforms including hTAF_{II}80δ (lane 2) **b**, The ratio of hTAF_{II}80δ mRNA with respect to that of hTAF_{II}80α increases in HL-60 undergoing all-trans retinoic acid (RA) induced apoptosis. RT-PCR products were obtained as in panel **a**, lane 2.

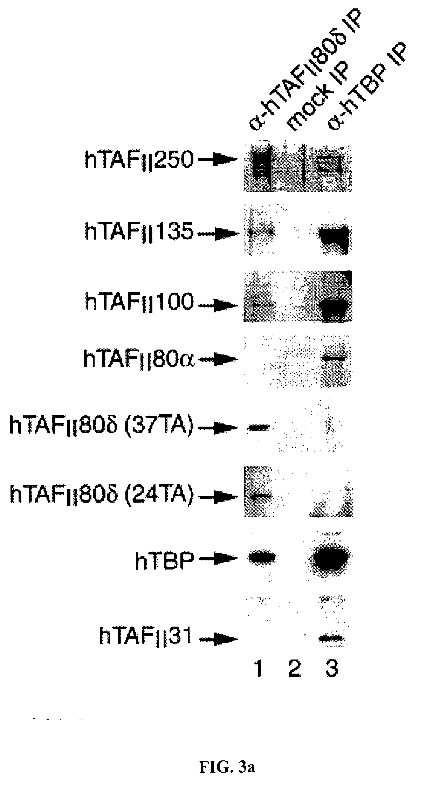

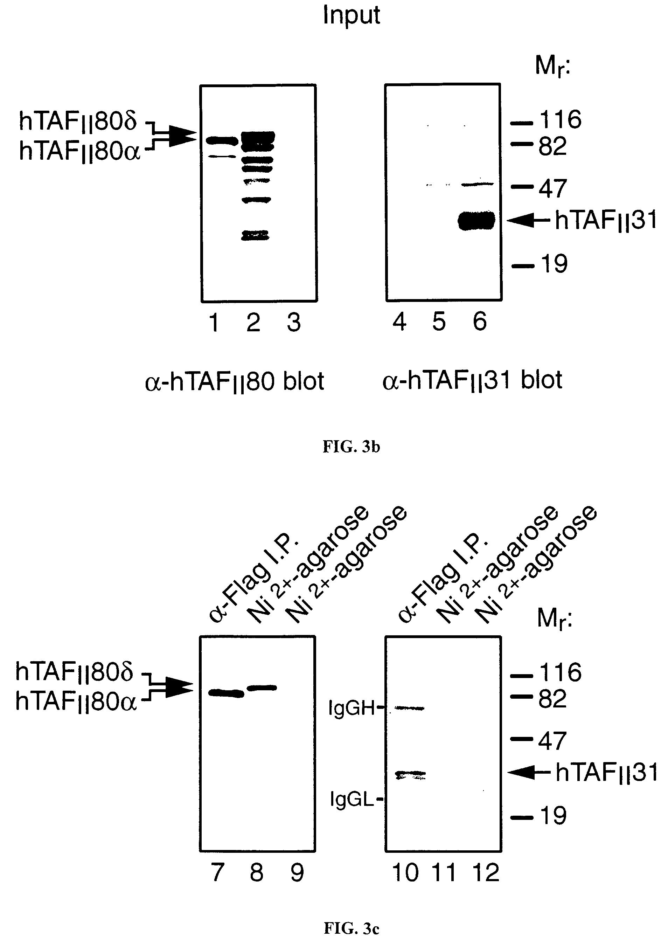

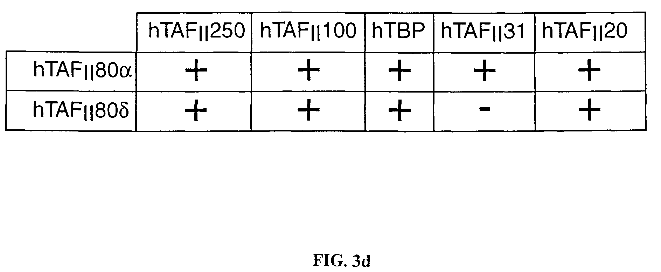

### Figure 3: hTAF_{II}80δ is present in a TFIID-like complex lacking hTAF_{II}31 in HeLa cells.

**a,** Immunoprecipitation (I.P.) of hTAF_{II}80δ and TBP-containing complexes from HeLa cells treated with etoposide VP-16 for 12 hours. Eluted complexes were concentrated 10-fold by trichloroacetic acid precipitation, fractionated by SDS-PAGE and electrophoretically transferred to nitrocellulose membranes. hTAF_{II} composition was determined by immunoblotting with appropriate antibodies. As a negative control, protein-G beads alone were incubated with HeLa extract and processed in the same manner as I.P.s (mock I.P.). The presence of two distinct epitopes of hTAF_{II}80δ were tested using mAb 24TA against the N-terminal Alu repeat derived sequence, and mAbs 37TA-1 and -3 both against the modified histone homology region. **b,** hTAF_{II}80δ is not present in TFTC and is not found complexed with GCN5. I.P.s and immunoblots were performed as in **a**, lane 3 contains purified TFTC (Wieczoreck et al, 1998). **c,** hTAF_{II}31 interacts *in vitro* with recombinant hTAF_{II}80α but not with hTAF_{II}80δ. Detection of recombinant TAF_{II}s from SF9 cells expressing Flag-hTAF_{II}80α (lane 1), His-hTAF_{II}80δ (lane 2) or (lane 3) by immuno-blotting with anti-TAF_{II}80 mAb and anti-TAF_{II}31 Abs. Flag-hTAF_{II}80α or His-hTAF_{II}80δ-containing extracts were mixed with hTAF_{II}31-containing extracts, followed by incubation with anti-Flag-agarose or Ni²⁺-agarose beads, respectively. After washing the beads, the amount of hTAF_{II}31 bound to immobilized Flag-hTAF_{II}80δ (lane 4) or His-hTAF_{II}80δ (lane 5) was analyzed by immunoblotting. **d,** A summary of protein-protein interactions observed between hTAF_{II}80δ and hTAF_{II}80α and other TAF_{II}s using co-expression in SF9 cells followed by co-immunoprecipitation.

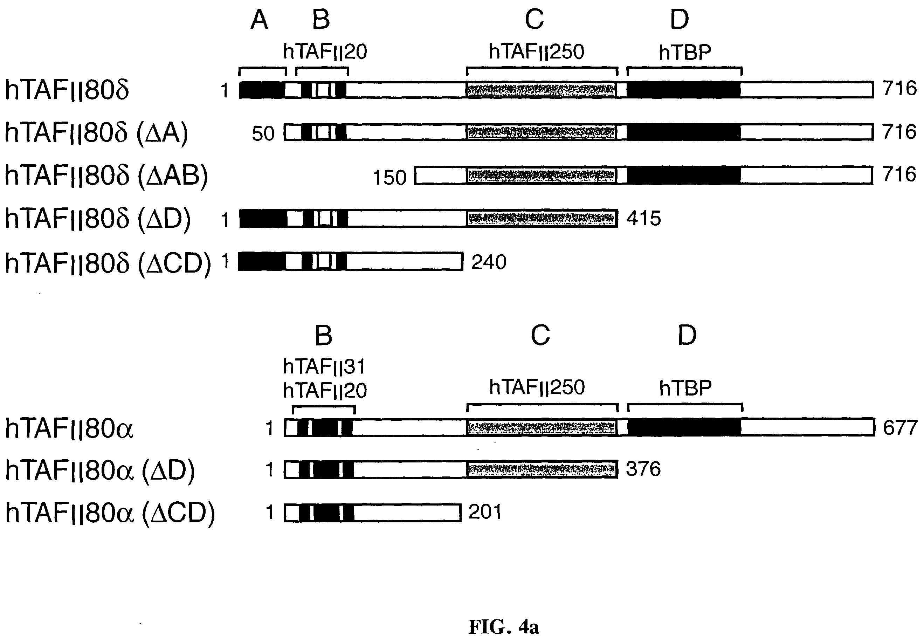

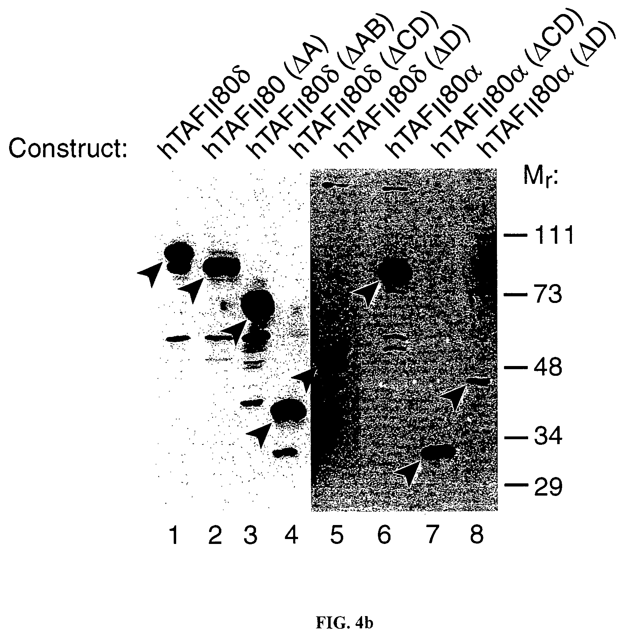

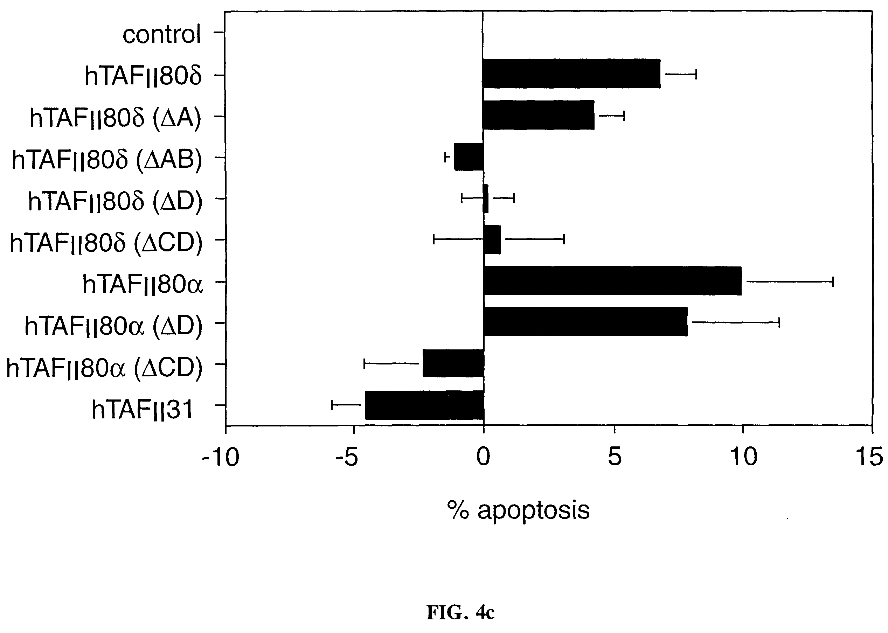

### Figure 4: Overexpression of hTAF_{II}80δ in HeLa cells causes apoptosis.

**a,** Schematic representation of the hTAF_{II}80α and hTAF_{II}80δ derivatives used in transient transfection assays. For simplicity, domains have been given letter codes. The hTAF_{II}s reported to interact with various portions of hTAF_{II}80 *in vitro* (Hisatake et al, 1995) are indicated. The amino acids expressed by each of the deletion variants are given at the left and right of the schema. The unique N-terminal tail of hTAF_{II}80δ is indicated by black shading, as are the α-helices of the histone fold motif. **b,** Immunoblot analysis of transfected deletion variants. Arrow heads indicate overexpressed proteins. **c**, Percentage apoptosis induced by transfection of hTAF_{II}s. The standard deviation of three independent transfections is given by error bars. Positive values indicate increased apoptosis and negative values decreased apoptosis.

### Figure 5: Alignment between hTAF_{II}80δ and the corresponding encoded amino acid sequence.

The ATG initiation codon and the first amino acid are in bold ; the locus of the deletion is in bold as well as the region surrounding it, which is useful for detecting specifically hTAF_{II}80δ.

### Example 1: Identification of a new hTAF_{II}80 isoform.

An oligo-dT primed HeLa cell cDNA λ ZAP-II phage library was screened with a random labelled cDNA fragment encoding the 340 C-terminal amino acids of the hTAF_{II}80α. Two positive phage clones were independently picked and subjected to sequence analysis. These two clones were identical and encoded the δ isoform of hTAF_{II}80 (figure 1). The gene and protein sequences are presented at figure 5. The ATG initiation codon and the first amino acid are in bold; the locus of the deletion is in bold as well as the region surrounding it which is particularly useful for detecting specifically hTAF_{II}80δ.

### Example 2: hTAF_{II}80δ protein expression is induced in apoptotic cells.

### Methods

### RT-PCR

Total RNA was isolated from cells using Trizol (GibcoBRL) according to the manufacturer's recommendations. RT-PCR reactions were performed as described (Metzger et al, 1999) for 20-30 cycles. The sequences of all oligonucleotides used in this study are available upon request.

### Antibodies

Antibodies were produced as previously described (Wieczorek et al, 1998) using the peptides: CSPSLHRGLHPSREEKRDSRM (SEQ ID N° 6) for mAb 24TA against the unique N-terminal tail of hTAF_{II}80δ; QLLTDEDALKFMHMGC (SEQ ID N° 7) for mAbs 37TA-1 and -3 against the unique altered H4 homolog region of hTAF_{II}80δ; and LTDEVSYRIKEIAQDALKC (SEQ ID N° 8) for polyclonal mouse Abs 25TA specific for α-helix 2 of the histone fold motif of hTAF_{II}80δ. Anti-hTAF_{II}31 antibodies were described by Martinez et al, 1998 (Fig. 3a) or Sant Cruz Biotechnology (Fig. 3b). In Fig. 4b, polyclonal rabbit anti-hTAF_{II}80 antibodies from Transduction Laboratories were used. All other antibodies against hTAF_{II}s have been previously described (Wieczorek et al, 1998 and Dantonel et al, 1997).

### Immunocytochemistry

HeLa cells grown on cover slips were treated with 100 µM etoposide VP-16 (BIOMOL) for 4 hours and then washed twice with phosphate buffered saline (PBS). Cells were fixed in a solution of 2% paraformaldehyde for 4 minutes. Cells were permeabilized by treatment with PBS- 0.1% Triton X-100 (PBS-T) twice for 5 minutes. Cells were then incubated with anti-hTAF_{II}80δ mAb 24TA diluted 1/500 in PBS-T for 2 hours at room temperature before washing three times with PBS-T. A CY3-labelled goat anti-mouse IgG secondary antibody (Jackson Laboratories) was added for 1 hour at room temperature followed by three washes in PBS-T. Fluorescein conjugated M30-CytoDEATH antibody (Roche) was then added at 1/250 in PBS-T and incubated further for 1 hour at room temperature. Cells were washed twice in PBS-T and once in PBS before the addition of Hoechst and visualization by immunofluorescent microscopy.

### Results

We first analyzed hTAF_{II}80δ expression by RT-PCR, using oligonucleotide primers that flank the region coding for the altered α-helix 2 of hTAF_{II}80δ which amplify products of distinct lengths from either hTAF_{II}80δ mRNA or mRNAs coding for its other histone-fold motif-containing isoforms (see Methods). Our initial experiments revealed low levels of hTAF_{II}80δ mRNA in logarithmically growing HeLa cells (Fig. 2a, lane 2) and HL-60 promeylocytic leukemia cells (Fig. 2b, lane 2). These results provided evidence that hTAF_{II}80δ mRNA is expressed.

When apoptosis is induced in human HL-60 cells by treatment with all-trans retinoic acid (Barnett et al, 1993) we observed an increase in the ratio of hTAF_{II}80δ with respect to total hTAF_{II}80 mRNA (Fig. 2b, lanes 3 and 4 versus 2). Thus, increased hTAF_{II}80δ mRNA levels correlated with cell death by apoptosis. An increased hTAF_{II}80δ/hTAF_{II}80α mRNA ratio was also observed in Jurkat T cells when apoptosis was induced by cycloheximide treatment, indicating that induction of hTAF_{II}80δ mRNA during apoptosis occurs in different cell lines exposed to distinct apoptotic stimuli.

Since hTAF_{II}80δ mRNA levels increase in populations of cells which are given apoptotic stimuli, we next determined whether the increased hTAF_{II}80δ mRNA levels resulted in a corresponding increase in hTAF_{II}80δ protein levels, and whether hTAF_{II}80δ expression correlated with apoptosis at the single cell level. Monoclonal antibodies were generated against two unique epitopes of hTAF_{II}80δ and a unique epitope of hTAF_{II}80δ corresponding to the intact α-helix 2 of its histone fold (see Fig. 1a and Methods). These antibodies were then used in immunocytochemistry experiments on HeLa cells treated with the apoptosis-inducing etoposide VP-16. To score for cells undergoing apoptosis, we co-stained cells with monoclonal antibodies that recognize a caspase cleavage product of cytokeratin 18 (Leers et al, 1999) As expected, apoptotic cells that stained positively for cleaved cytokeratin 18 were found to have a condensed nuclear morphogy by Hoechst staining. More importantly, all cells that stained positively for caspase activation also stained positively for hTAF_{II}80δ. In control experiments, all cells stained positively with antibodies that recognize all hTAF_{II}80 isoforms. These results show that hTAF_{II}80δ protein expression is indeed specifically induced in apoptotic cells.

### Example 3: hTAF_{II}80δ interaction with other TAFII proteins forms a new TFIID complex leading to apoptosis.

### Methods

### Immunoprecipitations

Immunoprecipitations (I.P.s) were performed as previously described (Wieczorek et al, 1998 and Jacq et al, 1994) with the exception that all antibodies were used directly as ascites fluids. For the immunoprecipitation of hTAF_{II}80δ, three anti-hTAF_{II}80δ were pooled (24TA, 37TA-1, 37TA-3). hTAF_{II}80δ-containing complexes were eluted by the addition of the corresponding peptides at 2.5 mg/ml. For the anti-TBP I.P. the 2C1 mAb was used (Jacq et al, 1994).

### Plasmids

The cDNAs for hTAF_{II}80δ, hTAF_{II}80α, and hTAF_{II}31 were subcloned into pVL1392 (Pharmingen) by PCR for baculovirus production. Plasmids expressing hTAF_{II}80δ and hTAF_{II}80α derivatives were constructed in the expression vector pXJ42 (Xiao et al, 1991) using a PCR-based approach. Constructions were verified by DNA sequence analysis.

### Protein-protein interaction assays

*In vitro* protein-protein interactions between FLAG-tagged hTAF_{II}80α and 6XHistagged hTAF_{II}80δ produced by Baculovirus overexpression were performed using either anti-FLAG M2 affinity gel (Sigma) or Ni-NTA resin (Qiagen), respectively. Whole cell extracts from infected SF9 cells overexpressing FLAG-tagged hTAF_{II}80α and 6XHis-tagged hTAF_{II}80δ were mixed with extracts containing hTAF_{II}31 and gently mixed for two hours at 4 °C. Resulting protein-protein complexes were washed and analyzed as previously described (Jacq et al, 1994). Pairwise interactions between hTAF_{II}s were studied using co-infection of SF9 insect cells followed by coimmunoprecipitations as in ref. (Dantonel et al, 1997).

### Results

We probed for unique biochemical properties of hTAF_{II}80δ that could underlie its specific induction in apoptotic cells. We used hTAF_{II}80δ-specific antibodies to immunoprecipitate endogenous hTAF_{II}80δ from HeLa cells. Immunoprecipitated protein complexes were eluted by the addition of excess cognate epitope peptides and analysed by immunoblotting experiments for TFIID components. Like the ubiquitously expressed hTAF_{II}80α, hTAF_{II}80δ is found in a TFIID-like complex containing hTAF_{II}250, hTAF_{II}135, hTAF_{II}100 and hTBP in HeLa cells (Fig. 3a). In contrast to hTAF_{II}80α, however, hTAF_{II}80δ does not co-immunoprecipitate with hTAF_{II}31 (Fig. 3a, lane 1). Moreover, hTAF_{II}80δ immunoprecipitates do not contain hTAF_{II}80α (Fig. 3a, lane 1), suggesting that incorporation of these two isoforms into distinct TFIID-like complexes is mutually exclusive. hTAF_{II}80δ can be found in TFIID as well as other TAF_{II}-containing complexes lacking TBP including TFTC (Wieczorek et al, 1998) and STAGA (Martinez et al, 1998). To determine if hTAF_{II}80δ could be found associated with TFTC or STAGA we tested hTAF_{II}80δ immunoprecipitates for the presence of the TFTC and STAGA specific subunit, GCN5 (Wieczorek et al, 1998 and Martinez et al, 1998). GCN5 does not co-immunoprecipitate with hTAF_{II}80δ (Fig. 3b, lane 1). Morevover, purified TFTC does not contain hTAF_{II}80δ (Fig 3b, lane 3). Thus, it appears that hTAF_{II}80δ functions as part of TFIID-like complexes but not in TFTC or STAGA. Since the histone fold region of hTAF_{II}80δ is disrupted, a simple explanation for the absence of hTAF_{II}31 in hTAF_{II}80δ-containing TFIID-like complexes would be a direct inability of hTAF_{II}80δ to contact hTAF_{II}31.
We tested this hypothesis directly using Baculovirus produced recombinant proteins (Fig. 3c). Recombinant hTAF_{II}80δ immobilized on agarose beads retained recombinant hTAF_{II}31 (Fig. 3c, lane 10), whereas hTAF_{II}80δ was severely impaired for interaction with hTAF_{II}31 (Fig. 3c, lane 10). Finally, we tested all of the known hTAF_{II}80 targets within TFIID for interactions with hTAF_{II}80δ using co-expression in SF9 cells followed by co-immunoprecipitation. hTAF_{II}250, hTAF_{II}100, hTBP, and hTAF_{II}20 were all able to interact with hTAF_{II}80δ (Fig. 3d). The hTAF_{II}80-hTAF_{II}20 interaction has been reported to depend on the histone fold region of hTAF_{II}80 (Bando et al, 1997) yet hTAF_{II}20 can efficiently interact with hTAF_{II}80δ, suggesting the modified histone homology region of hTAF_{II}80δ retains a surface that can interact with hTAF_{II}20. The above biochemical studies indicate that hTAF_{II}80δ is present in a novel complex resembling TFIID in cells, but due to the deletion in its histone fold motif, lacks the histone H3-like hTAF_{II}31. We term this hTAF_{II}80δ-containing TFIID-like complex TFIID π.

### Example 4: hTAF_{II}80δ overexpression is sufficient to trigger apoptosis.

### Methods

### Apotosis assays

HeLa cells were co-transfected by the calcium phosphate precipitation method using 1-2 µg of pBB14 (expressing Us9-GFP) (Kaleijta et al, 1999) together with a ten fold excess of each hTAF_{II} expression vector. 41 hours after transfection adherent and floating cells were pooled, washed in PBS and fixed in 70% ethanol. Fixed cells were treated with RNAse A at Img/ml and propidium iodide at 50 µg/ml in PBS were then added for at least 1 hour at room temperature. The DNA content of transfected cells was then analyzed by flow cytometry, gating for GFP-expressing cells as previously described (Kaleijta et al, 1999). The percentage apoptosis specifically induced by hTAF_{II} expression is given as the percentage of sub-G1 cells observed when the cells were co-transfected with a given TAF_{II}-expression vector minus that observed with co-transfection of the empty expression vector alone.

### Results

To address the function of TFIIDπ in apoptotic cells, we reasoned that artificial elevation of hTAF_{II}80δ levels in cells should compete with endogenous hTAF_{II}80α for incorporation into cellular TFIID, and thus drive the formation of TFIIDπ. We further hypothesized that if TFIIDπ is an important part of a death-inducing genetic program, the overexpression of hTAF_{II}80δ might be sufficient to trigger apoptosis. Transient transfection of HeLa cells was used to assay the effect of overexpression of different TFIID subunits. Various TAF_{II}-expressing plasmids (Fig. 4a) were co-transfected into HeLa cells together with a plasmid expressing membrane bound green fluorescent protein (GFP), which provides a convenient marker for transfected cells (Kaleijta et al, 1999). To measure apoptosis, the sub-G1 DNA content of cells was quantified by propidium iodide staining followed by flow cytometry (Ryan et al, 1998). In parallel, the expression of transfected TAF_{II}s was monitored by Western blotting of cell extracts (Fig. 4b). The transfection of hTAF_{II}80δ resulted in a significant increase in apoptosis (Fig. 4c). This increased apoptosis was specific to hTAF_{II}80δ, since the transfection of 10 other TFIID subunits under the same conditions showed no increase in apoptosis over a vector control.

### Example 5: hTAF_{II}80α overexpression is also sufficient to trigger apoptosis.

Surprisingly, however, the only other TFIID subunit whose overexpression triggered apoptosis was hTAF_{II}80α (Fig. 4c). One explanation for this observation is that hTAF_{II}80α overexpression might also drive the formation of a TFIID-like complex lacking hTAF_{II}31, by titrating hTAF_{II}31 away from the functional cellular pool of TFIID. To directly test whether apoptosis is sensitive to the intracellular level of hTAF_{II}31, we transfected hTAF_{II}31 into HeLa cells. hTAF_{II}31 overexpression repressed the background apoptosis resulting from the transfection protocol (Fig. 4c), suggesting that apoptosis is induced by elevated hTAF_{II}80δ or hTAF_{II}80α levels but antagonized by increased hTAF_{II}31 levels. These results together support the view that apoptosis can be induced by the exclusion of hTAF_{II}31 from TFIID.

### Example 6: the altered histone fold region is crucial for hTAF_{II}80δ's apoptotic function.

To further understand how hTAF_{II}80δ and hTAF_{II}80α overexpression contributes to apoptosis, plasmids expressing truncated variants of these proteins were transfected into HeLa cells. In the case of hTAF_{II}80δ, the removal of the unique N-terminal 49 amino acids causes a small but reproducible decrease in its ability to induce apoptosis [Fig. 4c, hTAF_{II}80δ (ΔA)], suggesting that although this portion of hTAF_{II}80δ contributes to its apoptotic function, it is not absolutely required. Removal of the entire histone fold region of hTAF_{II}80δ abolished its ability to induce apoptosis [Fig. 4c, hTAF_{II}80δ (ΔAB)], underlining the fact that the altered histone fold region is crucial for hTAF_{II}80δ's apoptotic function. C-terminal deletions of hTAF_{II}80δ that removed domains which are required to bind TBP or hTAF_{II}250 *in vitro,* do not induce apoptosis [Fig. 4c, hTAF_{II}80δ (ΔD and ΔCD)]. In contrast to hTAF_{II}80δ, hTAF_{II}80α was still able to induce apoptosis when the TBP binding domain was removed from hTAF_{II}80δ [Fig. 4c, compare hTAF_{II}80δ (ΔD) with hTAF_{II}80α (ΔD)]. Another difference between these two isoforms was that the expression of the histone fold of hTAF_{II}80α alone inhibited apoptosis while the corresponding region of hTAF_{II}80δ was essentially inert [Fig. 4c, compare hTAF_{II}80α (ΔCD) and hTAF_{II}80δ (ΔCD)]. The fact that the TBP binding domain of hTAF_{II}80α is dispensable for its pro-apoptotic activity further substantiates the hypothesis that hTAF_{II}80α overexpression triggers cell death by titrating hTAF_{II}31 away from active TFIID. The above deletion studies establish the altered histone fold region and the C-terminal TBP-binding domain of hTAF_{II}80δ as crucial for its pro-apoptotic function, and provide further support for a mechanism where hTAF_{II}80δ induces apoptosis by incorporation into a TFIID-like complex that lacks hTAF_{II}31 (TFIIDπ).

### REFERENCES

- Albright, S. & Tjian, R. TAFs revisited: more data reveal new twists and confirm old ideas. Gene 242, 1-13 (2000).
- Aoki, T. et al. Rat TAFII31 gene is induced upon programmed cell death in differentiated PC12 cells deprived of NGF. Biochem Biophys Res Commun 234, 230-234 (1997).
- Apone, L. M. et al. Broad, but not universal, transcriptional requirement for yTAFII17, a histone H3-like TAFII present in TFIID and SAGA. Mol Cell 2, 653-661 (1998).
- Bando, M., Ijuin, S., Hasegawa, S. & Horikoshi, M. The involvement of the histone fold motifs in the mutual interaction between human TAF(II)80 and TAF(II)22. J Biochem (Tokyo) 121, 591-597 (1997).
- Barnett, T. R., Drake, L. & Pickle, W. d. Human biliary glycoprotein gene:
   characterization of a family of novel alternatively spliced RNAs and their expressed proteins. Mol Cell Biol 13, 1273-1282 (1993).
- Bell, B. & Tora, L. Regulation of gene expression by multiple forms of TFIID and other novel TAFII-containing complexes. Exp Cell Res 246, 11-19 (1999).
- Birck, C. et al. Human TAF(II)28 and TAF(II)18 interact through a histone fold encoded by atypical evolutionary conserved motifs also found in the SPT3 family.
   Cell 94, 239-249 (1998).
- Burley, S. K. & Roeder, R. G. Biochemistry and structural biology of transcription factor IID (TFIID). Annu Rev Biochem 65, 769-799 (1996).
- Dantonel, J. C., Murthy, K. G., Manley, J. L. & Tora, L. Transcription factor TFIID recruits factor CPSF for formation of 3' end of mRNA. Nature 389, 399-402 (1997).
- Gangloff, Y. G. et al. The human TFIID components TAF(II)135 and TAF(II)20 and the yeast SAGA components ADA1 and TAF(II)68 heterodimerize to form histone-like pairs. Mol Cell Biol 20, 340-351 (2000).
- Green, M. R. TBP-associated factors (TAFIIs): multiple, selective transcriptional mediators in common complexes. Trends Biochem Sci 25, 59-63 (2000).
- Hisatake, K. et al. Evolutionary conservation of human TATA-binding-polypeptide-associated factors TAFII31 and TAFII80 and interactions of TAFII80 with other TAFs and with general transcription factors. Proc Natl Acad Sci U S A 92, 8195-8199 (1995).
- Hoffmann, A. et al. A histone octamer-like structure within TFIID. Nature 380, 356-359 (1996).
- Jacq, X. et al. Human TAFII30 is present in a distinct TFIID complex and is required for transcriptional activation by the estrogen receptor. Cell 79, 107-117 (1994).
- Jehn, B. M. & Osborne, B. A. Gene regulation associated with apoptosis. Crit Rev Eukaryot Gene Expr 7, 179-193 (1997).
- Kalejta, R. F., Brideau, A. D., Banfield, B. W. & Beavis, A. J. An integral membrane green fluorescent protein marker, Us9-GFP, is quantitatively retained in cells during propidium iodide-based cell cycle analysis by flow cytometry. Exp Cell Res 248, 322-328 (1999).
- Leers, M. P. et al. Immunocytochemical detection and mapping of a cytokeratin 18 neo- epitope exposed during early apoptosis [In Process Citation]. J Pathol 187, 567-572 (1999).
- Martinez, E., Kundu, T. K., Fu, J. & Roeder, R. G. A human SPT3-TAFII31-GCN5-L acetylase complex distinct from transcription factor IID [published erratum appears in J Biol Chem 1998 Oct 16;273(42):27755]. J Biol Chem 273, 23781-23785 (1998).
- Metzger, D., Scheer, E., Soldatov, A. & Tora, L. Mammalian TAF(II)30 is required for cell cycle progression and specific cellular differentiation programmes. Embo J 18, 4823-4834 (1999).
- Michel, B., Komarnitsky, P. & Buratowski, S. Histone-like TAFs are essential for transcription in vivo. Mol Cell 2, 663-673 (1998).
- Moqtaderi, Z., Keaveney, M. & Struhl, K. The histone H3-like TAF is broadly required for transcription in yeast. Mol Cell 2, 675-682 (1998).
- Ryan, K. M. & Vousden, K. H. Characterization of structural p53 mutants which show selective defects in apoptosis but not cell cycle arrest. Mol Cell Biol 18, 3692-3698 (1998).
- Sekiguchi, T. et al. Apoptosis is induced in BHK cells by the tsBN462/13 mutation in the CCG1/TAFII250 subunit of the TFIID basal transcription factor. Exp Cell Res 218, 490-498 (1995).
- Wang, S., Dibenedetto, A. J. & Pittman, R. N. Genes induced in programmed cell death of neuronal PC12 cells and developing sympathetic neurons in vivo. Dev Biol 188, 322-336 (1997).
- Weinzierl, R. O., Ruppert, S., Dynlacht, B. D., Tanese, N. & Tjian, R. Cloning and expression of Drosophila TAFII60 and human TAFII70 reveal conserved interactions with other subunits of TFIID. EMBO J 12, 5303-5309 (1993).
- White, E. Life, death, and the pursuit of apoptosis. Genes Dev 10, 1-15 (1996).
- Wieczorek, E., Brand, M., Jacq, X. & Tora, L. Function of TAF(II)-containing complex without TBP in transcription by RNA polymerase II. Nature 393, 187-191 (1998).
- Xiao, J. H., Davidson, I., Matthes, H., Gamier, J. M. & Chambon, P. Cloning, expression, and transcriptional properties of the human enhancer factor TEF-1.
   Cell 65, 551-568 (1991).
- Xie, X. et al. Structural similarity between TAFs and the heterotetrameric core of the histone octamer. Nature 380, 316-322 (1996).
- Yamit-Hezi, A. & Dikstein, R. TAFII105 mediates activation of anti-apoptotic genes by NF-kappaB. EMBO J 17, 5161-5169 (1998).

## Claims

1. Polypeptide comprising a sequence having at least 80% identity with hTAFII80δ of the sequence SEQ ID N°1 **characterized in that** it has a 10 amino acids deletion at position 43 to 52 of SEQ ID N°3 (hTAFII80), said polypeptide being capable of inducing apoptosis when expressed in mammalian cells.

2. Polypeptide comprising the sequence SEQ ID N° corresponding to the hTAFII80δ.

3. Isolated nucleic acid sequence coding for a polypeptide according to one of claim 1 and 2.

4. Isolated nucleic acid sequence coding for hTAFII80δ having the sequence SEQ ID No. 1, said hTAFII80δ protein being an isoform of hTAFII80 comprising a deletion of the 10 amino acids at position 43 to 52 of hTAFII80 and being capable of inducing apoptosis when expressed in mammal cells.

5. Isolated nucleic acid sequence according to claim 4 having a sequence comprising the sequence SEQ ID N°2.

6. Complementary sequence of the nucleic sequence according to claims 3 to 5.

7. Isolated nucleic acid sequence, **characterized in that** it is selected from the group consisting of:
a) a fragment of a nucleic acid comprising at least 12 consecutive bases preferably of a sequence according to one of claims 3 to 6 present at the junction of the deletion, more particularly a sequence encompassing G789 and G790 of the sequence depicted at figure 5,
b) a nucleic acid sequence capable of hybridizing specifically with the nucleic acid sequence as defined in a) and comprising at least 12 bases.

8. Nucleic acid sequence according to claim 7 as a primer or a probe.

9. Nucleic acid sequence according to claims 7 and 8, **characterized in that** it comprises a sequence selected from the group consisting of sequences SEQ ID No. 4 and SEQ ID No 5.

10. Cloning and/or expression vector comprising a nucleic acid sequence according to one of claims 3 to 6.

11. Vector according to claim 10 further comprising the elements allowing the expression and/or secretion of the said sequences in a host cell.

12. Host cell transformed by a vector according to one of claims 10 and 11 which is an eukaryotic or a prokaryotic cell.

13. Mammal, except human, **characterized in that** it comprises a cell according to claim 12.

14. Mono- or polyclonal antibodies or fragments thereof, chimeric or immunoconjugated antibodies, **characterized in that** they are capable of specifically recognizing a polypeptide according to one of claims 1 to 2.

15. Method for the diagnosis of a pathology associated with the expression or the lack of expression of hTAFII80, hTAFII80 isoforms, notably hTAFII80δ **characterized in that** one or more antibodies according to claim 14 is(are) brought into contact with the biological material to be tested, under conditions allowing the possible formation of specific immunological complexes between the said polypeptide and the said antibody or antibodies, and **in that** the immunological complexes possibly formed are detected.

16. Method for the determination of an allelic variability or a loss of heterozygosity, **characterized in that** it uses a nucleic acid sequence according to one of claims 7 to 9.

17. Method for determining if a subject is at increased risk of developing a pathology associated with the expression or the lack of expression of hTAFII80, hTAFII80 isoforms, notably hTAFII808, comprising the steps consisting of:
a) collecting a biological sample containing genomic DNA or RNA from the subject;
b) testing for the presence of mutations, polymorphism, or determining the sequence, or length thereof, of a fragment of said DNA or RNA susceptible of containing a mutation or a polymorphism associated with the risk of developing cancer and/or a pathology associated with the expression of hTAFII80, hTAFII80 isoforms, notably hTAFII80δ, said fragment being amplified by polymerase chain reaction with a set of primers that hybridizes to a hTAFII80 or isoforms thereof sequence, notably primers derived from SEQ ID N°1 and/or SEQ ID N°3, such as the nucleic acid according to one of the claims 7 to 9;
c) determining whether or not the subject is at increased risk developing cancer and/or a pathology associated with the expression or the lack of expression of hTAFII80, hTAFII80 isoforms, notably hTAFII80δ by observing if the sequence of said fragment of DNA or RNA contains a mutation or polymorphism, the presence of said mutation or polymorphism being an indication that said subject is at increased risk of developing cancer and/or said pathology.

18. A method according to anyone of claims 15 to 17, wherein the pathology associated with the expression or the lack of expression of hTAFII80, hTAFII80 isoforms, notably hTAFII80δ is selected from the group consisting of cell apoptosis, notably nerve cell degeneration, such as Alzheimer disease and Parkinson disease; cancer and AIDS.

19. Kit for diagnostic or for determining if a subject is at increased risk of developing cancer and/or a pathology associated with the expression or the lack of expression of hTAFII80, hTAFII80 isoforms, notably hTAFII80δ, comprising at least one pair of primers capable of amplifying a fragment of genomic DNA or RNA encoding the protein hTAFII80, hTAFII80 isoforms, notably hTAFII80δ, preferably said primers being chosen among primers derived from SEQ ID N°1, such as the nucleic acid according to one of the claims 7 to 9.

20. Use of a cell according to claim 12, of a mammal according to claim 13, or of a vector according to one of claims 10 and 11, for assessing the expression and/or the activity of the protein hTAFII80, hTAFII80 isoforms, notably hTAFII80δ, for studying the direct or indirect interactions between said proteins with other TAF proteins, more particularly with hTAFII31, and for selecting compounds that modulate the activity and/or expression of said protein.

21. Use according to claim 20 for screening compounds capable of interacting directly or indirectly with hTAFII80 or hTAFII80 isoforms, notably with hTAFII80δ, and/or capable of modulating the expression or the activity of said proteins.

22. Process for selecting compounds capable of modulating the activity of hTAFII80 and/or hTAFII80 isoforms, notably hTAFII80δ **characterized in that** it comprises the step consisting of:
a) overexpressing hTAFII80 or hTAFII80 isoforms, notably hTAFII80δ in host cells,
b) growing said cells in a medium containing at least one compound to be tested,
c) measuring the rate of apoptosis compared to cells grown in compound-free medium,
d) selecting compounds that reduce or increase significantly the rate of apoptosis.

23. Compound obtainable by the process according to claim 22.

24. Compound according to claim 23, **characterized in that**:
a) it decreases significantly the level of hTAFII80 or hTAFII80 isoforms, notably hTAFII80δ protein expression;
b) it increases by at least 50% the rate of apoptosis induced by the expression of said proteins; or
c) it decreases by at least 50% the rate of apoptosis induced by the expression of said proteins.

25. Compound according to one of claims 23 and 24, **characterized in that** it is chosen from:
a) an antibody according to claim 14;
b) a peptide or a peptide like molecule;
c) a vector according to one of claims 10 and 11;
d) a sense or anti-sense nucleic comprising a sequence able to hybridize to a nucleic acid according to claim 1 to 3 or to SEQ ID N°3; or
e) a chemical.

26. Use of a compound according to one of claims 23 to 25, for preparing a drug.

27. Use according to claim 26 for preparing a drug for treating diseases involving cell apoptosis, notably nerve cell degeneration, such as Alzheimer disease and Parkinson disease.

28. Use according to claim 26 for preparing a drug for treating cancer.

29. Use according to claim 26 for preparing a drug for treating AIDS.

30. Use of a nucleic acid sequence coding for a hTAFII80 protein or isoforms thereof for inducing apoptosis.

31. Use of a vector expressing a hTAFII80 protein or isoforms thereof for inducing apoptosis.

32. Use of a fragment of at least 12 bases of a sequence derived from a nucleic acid sequence coding for a hTAFII80 protein or isoforms thereof as an anti-sense oligonucleotide for inhibiting apoptosis in a cell.

33. Use of a fragment of at least 12 bases of a sequence derived from a nucleic acid sequence coding for a hTAFII80 protein or isoforms thereof as a probe or a primer for the diagnostic of cell apoptosis, notably nerve cells degeneration, such as Alzheimer disease and Parkinson disease; cancer and AIDS or for determining if a subject is at increased risk of developing such diseases.

34. Use of a compound capable of inhibiting the expression or activity of hTAFII80 protein or isoforms thereof for preparing a drug.

35. Use of a compound capable of activating the expression or activity of hTAFII80 protein or isoforms thereof for preparing a drug.

36. Use of a compound capable of interfering directly or indirectly with the hTAFII80 protein or isoforms thereof interaction with other transcription associated factor proteins, preferably with other hTAFII proteins such as hTAFII31, for preparing a drug.

37. Use according to claim 34 or 36 for preparing a drug for treating diseases involving cell apoptosis, notably nerve cells degeneration, such as Alzheimer disease and Parkinson disease, cancer and AIDS.


SEQUENCE LISTING

<110> INSTITUT NATIONAL DE LA SANTÉ ET DE LA RECHERCHE MÉDICALE - INSERM

<120> USE OF hTAF_{II}80 AND ISOFORMS THEREOF FOR INDUCING APOPTOSIS

<130> D18842

<160> 8

<170> PatentIn Ver. 2.1

<210> 1
<211> 667
<212> PRT
<213> Homo sapiens

<220>
<223> hTAF_{II}80δ

<400> 1
Met Ala Glu Glu Lys Lys Leu Lys Leu Ser Asn Thr Val Leu Pro Ser
  1               5                  10                  15

Glu Ser Met Lys Val Val Ala Glu Ser Met Gly Ile Ala Gln Ile Gln
             20                  25                  30

Glu Glu Thr Cys Gln Leu Leu Thr Asp Glu Asp Ala Leu Lys Phe Met
         35                  40                  45

His Met Gly Lys Arg Gln Lys Leu Thr Thr Ser Asp Ile Asp Tyr Ala
     50                  55                  60

Leu Lys Leu Lys Asn Val Glu Pro Leu Tyr Gly Phe His Ala Gln Glu
 65                  70                  75                  80

Phe Ile Pro Phe Arg Phe Ala Ser Gly Gly Gly Arg Glu Leu Tyr Phe
                 85                  90                  95

Tyr Glu Glu Lys Glu Val Asp Leu Ser Asp Ile Ile Asn Thr Pro Leu
            100                 105                 110

Pro Arg Val Pro Leu Asp Val Cys Leu Lys Ala His Trp Leu Ser Ile
        115                 120                 125

Glu Gly Cys Gln Pro Ala Ile Pro Glu Asn Pro Pro Pro Ala Pro Lys
    130                 135                 140

Glu Gln Gln Lys Ala Glu Ala Thr Glu Pro Leu Lys Ser Ala Lys Pro
145                 150                 155                 160

Gly Gln Glu Glu Asp Gly Pro Leu Lys Gly Lys Gly Gln Gly Ala Thr
                165                 170                 175

Thr Ala Asp Gly Lys Gly Lys Glu Lys Lys Ala Pro Pro Leu Leu Glu
            180                 185                 190

Gly Ala Pro Leu Arg Leu Lys Pro Arg Ser Ile His Glu Leu Ser Val
        195                 200                 205

Glu Gln Gln Leu Tyr Tyr Lys Glu Ile Thr Glu Ala Cys Val Gly Ser
    210                 215                 220

Cys Glu Ala Lys Arg Ala Glu Ala Leu Gln Ser Ile Ala Thr Asp Pro
225                 230                 235                 240

Gly Leu Tyr Gln Met Leu Pro Arg Phe Ser Thr Phe Ile Ser Glu Gly
                245                 250                 255

Val Arg Val Asn Val Val Gln Asn Asn Leu Ala Leu Leu Ile Tyr Leu
            260                 265                 270

Met Arg Met Val Lys Ala Leu Met Asp Asn Pro Thr Leu Tyr Leu Glu
        275                 280                 285

Lys Tyr Val His Glu Leu Ile Pro Ala Val Met Thr Cys Ile Val Ser
    290                 295                 300

Arg Gln Leu Cys Leu Arg Pro Asp Val Asp Asn His Trp Ala Leu Arg
305                 310                 315                 320

Asp Phe Ala Ala Arg Leu Val Ala Gln Ile Cys Lys His Phe Ser Thr
                325                 330                 335

Thr Thr Asn Asn Ile Gln Ser Arg Ile Thr Lys Thr Phe Thr Lys Ser
            340                 345                 350

Trp Val Asp Glu Lys Thr Pro Trp Thr Thr Arg Tyr Gly Ser Ile Ala
        355                 360                 365

Gly Leu Ala Glu Leu Gly His Asp Val Ile Lys Thr Leu Ile Leu Pro
    370                 375                 380

Arg Leu Gln Gln Glu Gly Glu Arg Ile Arg Ser Val Leu Asp Gly Pro
385                 390                 395                 400

Val Leu Ser Asn Ile Asp Arg Ile Gly Ala Asp His Val Gln Ser Leu
                405                 410                 415

Leu Leu Lys His Cys Ala Pro Val Leu Ala Lys Leu Arg Pro Pro Pro
            420                 425                 430

Asp Asn Gln Asp Ala Tyr Arg Ala Glu Phe Gly Ser Leu Gly Pro Leu
        435                 440                 445

Leu Cys Ser Gln Val Val Lys Ala Arg Ala Gln Ala Ala Leu Gln Ala
    450                 455                 460

Gln Gln Val Asn Arg Thr Thr Leu Thr Ile Thr Gln Pro Arg Pro Thr
465                 470                 475                 480

Leu Thr Leu Ser Gln Ala Pro Gln Pro Gly Pro Arg Thr Pro Gly Leu
                485                 490                 495

Leu Lys Val Pro Gly Ser Ile Ala Leu Pro Val Gln Thr Leu Val Ser
            500                 505                 510

Ala Arg Ala Ala Ala Pro Pro Gln Pro Ser Pro Pro Pro Thr Lys Phe
        515                 520                 525

Ile Val Met Ser Ser Ser Ser Ser Ala Pro Ser Thr Gln Gln Val Leu

530                 535                 540

Ser Leu Ser Thr Ser Ala Pro Gly Ser Gly Ser Thr Thr Thr Ser Pro
545                 550                 555                 560

Val Thr Thr Thr Val Pro Ser Val Gln Pro Ile Val Lys Leu Val Ser
                565                 570                 575

Thr Ala Thr Thr Ala Pro Pro Ser Thr Ala Pro Ser Gly Pro Gly Ser
            580                 585                 590

Val Gln Lys Tyr Ile Val Val Ser Leu Pro Pro Thr Gly Glu Gly Lys
        595                 600                 605

Gly Gly Pro Thr Ser His Pro Ser Pro Val Pro Pro Pro Ala Ser Ser
    610                 615                 620

Pro Ser Pro Leu Ser Gly Ser Ala Leu Cys Gly Gly Lys Gln Glu Ala
625                 630                 635                 640

Gly Asp Ser Pro Pro Pro Ala Pro Gly Thr Pro Lys Ala Asn Gly Ser
                645                 650                 655

Gln Pro Asn Ser Gly Ser Pro Gln Pro Ala Pro
            660                 665

<210> 2
<211> 2148
<212> DNA
<213> Homo sapiens

<220>
<223> hTAF_{II}80δ

<400> 2
atgacacaga tctttgctca ctgcaacctc cgcctcccgg gttcgagtga ttcttctgcc 60
tcagcctccc gagtagctgt gattacaggg tctccgtctc tccaccgggg gcttcatcct 120
tccagggagg agaagaggga ctccagaatg gctgaggaga agaagctgaa gcttagcaac 180
actgtgctgc cctcggagtc catgaaggtg gtggctgaat ccatgggcat cgcccagatt 240
caggaggaga cctgccagct gctaacggat gaggatgcct tgaagttcat gcacatgggg 300
aagcggcaga agctcaccac cagtgacatt gactacgcct tgaagctaaa gaatgtcgag 360
ccactctatg gcttccacgc ccaggagttc attcctttcc gcttcgcctc tggtgggggc 420
cgggagcttt acttctatga ggagaaggag gttgatctga gcgacatcat caatacccct 480
ctgccccggg tgcccctgga cgtctgcctc aaagctcatt ggctgagcat cgagggctgc 540
cagccagcta tccccgagaa cccgccccca gctcccaaag agcaacagaa ggctgaagcc 600
acagaacccc tgaagtcagc caagccaggc caggaggaag acggacccct gaagggcaaa 660
ggtcaagggg ccaccacagc cgacggcaaa gggaaagaga agaaggcgcc gcccttgctg 720
gagggggccc ccttgcgact gaagccccgg agcatccacg agttgtctgt ggagcagcag 780
ctctactaca aggagatcac cgaggcctgc gtgggctcct gcgaggccaa gagggcggaa 840
gccctgcaaa gcattgccac ggaccctgga ctgtatcaga tgctgccacg gttcagtacc 900
tttatctcgg agggggtccg tgtgaacgtg gttcagaaca acctggccct actcatctac 960
ctgatgcgta tggtgaaagc gctgatggac aaccccacgc tctatctaga aaaatacgtc 1020
catgagctga ttccagctgt gatgacctgc atcgtgagca gacagttgtg cctgcgacca 1080
gatgtggaca atcactgggc actccgagac tttgctgccc gcctggtggc ccagatctgc 1140
aagcatttta gcacaaccac taacaacatc cagtcccgga tcaccaagac cttcaccaag 1200
agctgggtgg acgagaagac gccctggacg actcgttatg gctccatcgc aggcttggct 1260
gagctgggac acgatgttat caagactctg attctgcccc ggctgcagca ggaaggggag 1320
cggatccgca gtgtgctgga cggccctgtg ctgagcaaca ttgaccggat tggagcagac 1380
catgtgcaga gcctcctgct gaaacactgt gctcctgttc tggcaaagct gcgcccaccg 1440
cctgacaatc aggacgccta tcgggcagaa ttcgggtccc ttgggcccct cctctgctcc 1500

caggtggtca aggctcgggc ccaggctgct ctgcaggctc agcaggtcaa caggaccact 1560
ctgaccatca cgcagccccg gcccacgctg accctctcgc aggccccaca gcctggccct 1620
cgcacccctg gcttgctgaa ggttcctggc tccatcgcac ttcctgtcca gacactggtg 1680
tctgcacgag cggctgcccc accacagcct tcccctcctc caaccaagtt tattgtaatg 1740
tcatcgtcct ccagcgcccc atccacccag caggtcctgt ccctcagcac ctcggccccc 1800
ggctcaggtt ccaccaccac ttcgcccgtc accaccaccg tccccagcgt gcagcccatc 1860
gtcaagttgg tctccaccgc caccaccgca ccccccagca ctgctccctc tggtcctggg 1920
agtgtccaga agtacatcgt ggtctcactt cccccaacag gggagggcaa aggaggcccc 1980
acctcccatc cttctccagt tcctcccccg gcatcgtccc cgtccccact cagcggcagt 2040
gccctttgtg gggggaagca ggaggctggg gacagtcccc ctccagctcc agggactcca 2100
aaagccaatg gctcccagcc caactccggc tcccctcagc ctgctccg 2148

<210> 3
<211> 677
<212> PRT
<213> Homo sapiens

<220>
<223> hTAF_{II}80

<400> 3


<210> 4
<211> 8
<212> DNA
<213> Artificial Sequence

<220>
<223> Description of Artificial Sequence: Preferred hTAFII80δ probe.

<400> 4
tgaggatg                                                                8


<210> 5
<211> 8
<212> DNA
<213> Artificial Sequence

<220>
<223> Description of Artificial Sequence: Preferred hTAFII80δ probe.

<400> 5
catcctca                                                                8


<210> 6
<211> 21
<212> PRT
<213> Homo sapiens

<220>
<223> Pepetide recognized by mAb 24TA (N-terminal tail of hTAFII80δ).

<400> 6
Cys Ser Pro Ser Leu His Arg Gly Leu His Pro Ser Arg Glu Glu Lys
1               5                   10                  15

Arg Asp Ser Arg Met
            20

<210> 7
<211> 16
<212> PRT
<213> Homo sapiens

<220>
<223> Peptide recognized by mAbs 37TA-1 and -3 against
      the unique altered H4 homolog region of hTAF_{II}80δ.

<400> 7
Gln Leu Leu Thr Asp Glu Asp Ala Leu Lys Phe Met His Met Gly Cys
1               5                   10                  15

<210> 8
<211> 19
<212> PRT
<213> Homo sapiens

<220>
<223> Peptide recognized by Abs 25TA specific for α-helix
      2 of the histone fold motif of hTAF_{II}80δ.

<400> 8
Leu Thr Asp Glu Val Ser Tyr Arg Ile Lys Glu Ile Ala Gln Asp Ala
1               5                   10                  15

Leu Lys Cys